# EUROPEAN PATENT SPECIFICATION

(11) **EP 1 018 008 B1**
(45) Date of publication and mention of the grant of the patent: **09.03.2005**
(21) Application number: 98950081.4
(22) Date of filing: 25.09.1998
(51) Int. Cl.: G01N 33/53

(54) **METHOD OF IDENTIFICATION OF LEADS OR ACTIVE COMPOUNDS**
VERFAHREN ZUR IDENTIFIZIERUNG VON LEITSTRUKTUREN ODER AKTIVEN VERBINDUNGEN
PROCEDE D'IDENTIFICATION DE COMPOSES SELECTIONNES APRES AU MOINS DEUX ETAPES DE CRIBLAGE OU DE COMPOSES ACTIFS

(30) Priority: 25.09.1997 EP 97402239
(43) Date of publication of application: 12.07.2000
(73) Proprietor: Cerep, 92500 Rueil-Malmaison (FR)
(72) Inventor: JEAN, Thierry, F-75013 Paris (FR); CHAPELAIN, Béatrice, F-86000 Poitiers (FR)
(74) Representative: Becker, Philippe
(86) International application number: PCT/EP1998/006105
(87) International publication number: WO 1999/015894

(56) References cited:
- WO-A-94/04552
- WO-A-95/30642
- WO-A-96/21859
- WO-A-96/30849
- WO-A-96/35953
- WO-A-97/15586
- N. K. TERRETT ET AL.: "Combinatorial synthesis-The design of compound libraries and their application to drug discovery." TETRAHEDRON., vol. 51, no. 30, 1995, pages 8135-8173, XP000644580 OXFORD GB cited in the application
- Journal of Immunological Methods 1996, 193, 2, 189 - 1997
- Molecular Diversity 1996, 1, 4, 233 - 240

## Description

The invention relates to methods of selection of leads or active compounds from libraries. More specifically, the invention provides methods of selection of leads or compounds which are active and selective towards a desired target. The instant invention can be used for instance in drug discovery processes.

The current methodologies in drug discovery involves the screening of large quantities of compounds of natural origin or obtained through combinatorial chemistry methods. Such methodologies are described, for instance, in "the Journal of Immunological Methods" (1996,193, 2, 189-197) and in "Molecular diversity" (1996, 1, 4, 233-240).

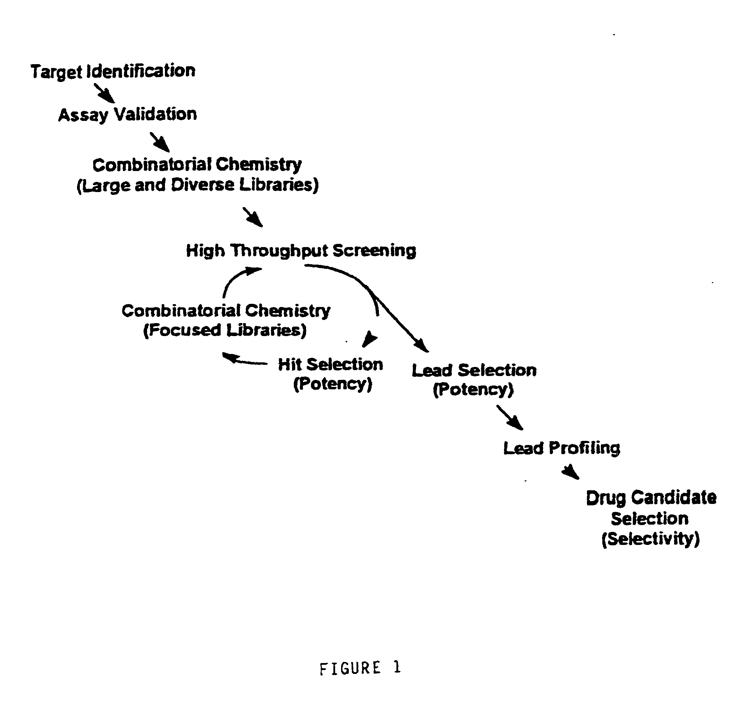

Libraries of several tens or hundreds of thousand of compounds are commonly tested in a single program of a drug discovery (figure 1). The selected compounds, namely hits, may be very numerous. The average hit rate is considered to be 0.1 %. Thus the screening of a 100,000 compounds library is likely to generate 100 hits. From the hit to the drug candidate is a long process involving multiple cycles of synthesis and screening of focused chemical libraries. This process of lead optimization is very time consuming and requires large budgets.

Indeed, hits to be followed up are commonly selected on the basis of the potency of their activity on the target screened. Each lead optimization cycle should allow to generate more active compounds. When a few hits exhibit the expected potency, usually after several optimization cycle, they become leads which are then tested for their selectivity against a large number of biological targets ("lead profiling"). The drug candidates are retained on the basis of their affinity for the chosen target and their specificity for this target (i.e. they should not be active on targets unrelated to the pathology object of the program). It is not rare that the selected leads prove to be unspecific, being thus likely to cause undesired effects. It is then necessary to start over the process from a less potent hit.

The present invention relates to improved methods of identification of leads or active compounds from libraries. The invention also relates to a high throughput profiling method, which allows the screening of selective compounds from large and diverse compositions. The invention also provides novel focused libraries comprising a plurality of compounds with structural diversity, but common functional properties.

The methods of the instant invention involve more specifically the determination of hit specificity ("profiling") at a very early stage in the drug discovery process. These new methods allow the identification of leads with improved properties (i.e. combining the criteria of potency and selectivity towards a given target) which can, optionally, be used in lead optimization processes to identify drug candidates with a higher predictability and lower costs.

It is therefore an object of the present invention to provide a method for the identification of leads from a library of compounds, said method comprising the steps of :
(a) screening a library of compounds for activity against a desired target, to obtain potent hits;
(b) profiling the potent hits of step (a) to obtain potent and selective leads.

As explained above, previous methods of drug discovery or lead selection involve a screening of a library of compounds for activity against a desired target, and the hits thereby obtained are then further processed through a lead optimization cycle to produce the leads. However, these steps are only based on the screening of libraries or focused libraries for a desired target, i.e. on the selection of compounds having the capacity to interact with a given target. None of the previous methods, however, involves a profiling step according to the instant invention. The high throughput screening method of the instant invention provides such a profiling step of the library so that the leads obtained combine both criteria of potency (capacity to interact with a given target) and selectivity (low or lack of interaction with other targets).

It is another object of the present invention to provide a method for the identification of active compounds from a library of compounds, said method comprising :
(i) a high throughput screening comprising the steps of :
   (a) screening a library of compounds for activity against a desired target, to obtain potent hits;
   (b) profiling the potent hits of step (a) to obtain potent and selective leads;
   (c) preparing focused library(ies) of compounds structurally related to the leads of step (b);
(ii) optionally repeating, one or several times, step (i) using the focused library(ies) of step (c) to generate further focused libraries, and,
(iii) screening, and preferably profiling, the focused libraries of step (i) or (ii) for said target, to obtain active, and preferably selective, compounds.

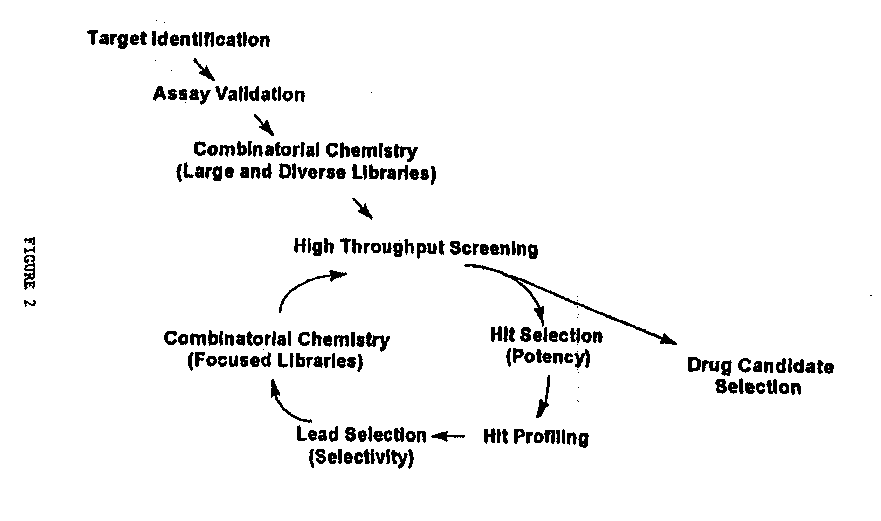

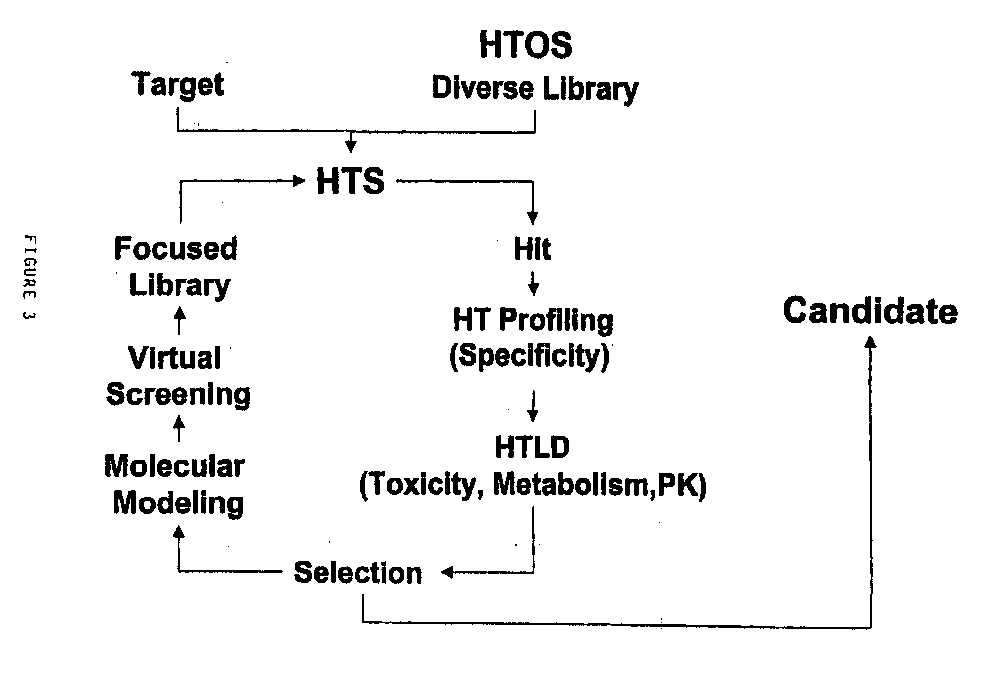

This method is illustrated in Figure 2 and comprises both the sequential steps of the lead identification method as disclosed above and the lead optimization steps, which produce active compounds.

For sake of clarity, the term "hit" designates, within the meaning of the instant invention, any compound selected after one screening step of a primary library. A hit is therefore a compound obtained after the first level of selection, which thus has at least the minimum potency vis-a-vis a desired target. A "lead" is an optimized hit, i.e. a hit whose activity has been confirmed, more preferably using a different selection test. Leads are therefore compounds obtained after at least 2 screening steps. Leads represent starting material for further optimization steps comprising the design of focused libraries, selection of active compounds, chemical modification thereof, functional testing, etc., which will produce drug candidate(s).

In the methods of the instant invention, a library is screened for activity against a chosen target. In general terms, the target can be any molecule such as for instance a receptor, a protein, a portion of a receptor, an antibody, a ligand, an infectious particle such as a virus, a bacteria, a fungus or a nucleic acid, etc., either in the pharmaceutical or in the agrochemical domain. More preferably, the target is a molecule involved in a biological or pathological pathway, such as a receptor, an enzyme, a nucleic acid region, an ion channel, etc. Particular types of targets are for instance receptors, such as those recited in Table I, and enzymes, such as those recited in Table II.

As indicated above, step (a) of the claimed methods comprises the screening of a library for activity against a given target. The screening for activity against a given target relates, in a general way, to the selection of any compounds of the library having the capacity to interact with said target. Within the context of the instant invention, the term "interaction" means either physical or functional interaction. More preferably, in step (a) of the method, the screening for compounds having activity against a desired target comprises the screening (or selection) of compounds capable of binding to the desired target, modifying the activity of an enzyme, or regulating the expression of a gene.

In a particular embodiment of the invention, step (a) comprises the screening for compounds that bind to a target. In a more preferred embodiment, the capacity of a compound to bind to a given target is measured by the capacity of said compound to inhibit the binding, to said target, of a reference, labelled molecule. As an example, the capacity of a compound to bind to a given target receptor can be measured by the capacity of said compound to inhibit the binding, to said target receptor, of a labelled ligand of said receptor. In this particular case, the screening therefore comprises, for each compound, the determination of the percentage of inhibition of the binding to the target of a labelled ligand. The capacity of a compound to bind to a given target can also be measured in a cell-based assay. In particular, the binding can be assessed by measuring, with appropriate markers, the activity induced by said compound within the cell.

In another particular embodiment of the invention, step (a) comprises the screening for compounds capable of modifying the activity of an enzyme. Said modification includes the stimulation or inhibition of the activity of said enzyme, or the alteration of the specificity or regulation of the activity of said enzyme, for instance.

The activity of the compound with respect to the target can be assayed according to various methods known in the art, such as enzymatic (in particular immunoenzymatic) methods, fluorescence-based detection methods, or radiassays, for instance. As particular suitable examples of fluorescence-based detection techniques, one can mention fluorescence polarization, fluorescence-correlation spectroscopy (Eigen et al., PNAS 91 (1994) 5740) and time-resolved fluorescence, for instance. Preferred examples of such techniques include for instance PSA (Baum et al., Anal.Biochem. 237 (1996) 129), HTRF (Mathis G., Clin. Chem. 39 (1993) 1953). As suitable examples of radio/immunoassays, it is convenient to use within the context of the present invention, ELISA or IRA for instance.

Said methods can be carried out in in vitro systems or in cell-based assays, as described in the experimental section. In particular, these assays are generally performed in containers such as plates (microwell plates), which are loaded with the appropriate reagents (target, buffer, test compound). Appropriate plates include 96-wells plates, as well as plates having a higher number of wells, in particular 384, 864, 1536 or more. Such plates are commercially available.

In order for this screening method to be more accurate, it is furthermore preferred that the activity of the compounds with respect to the target be confirmed. Confirmation may be obtained, for instance, by simply repeating the above screening method, but using the compound in isolated or purified form rather than contained in the whole library.

Depending on the size of the starting library, the number of confirmed hits may vary between 5 to 500, usually between 5 to 100.

Furthermore, in a preferred embodiment, the screening of step (a) further comprises measuring the affinity of the compounds capable of binding to said desired target, in order to select, as potent hits, the compounds having the best affinity. In order to determine the affinity, the confirmed hits are usually re-synthesized and purified. The affinity is then determined by methods known in the art, such as by "Scatchard" analysis as disclosed in the examples. Also, during this step, compounds can further be discarded because of their chemical structure.

Compounds obtained by the screening according to step (a), generally between 5 to 500, are then subjected to step (b). The profiling step (b) comprises in general terms assaying the potent hits on various targets and selecting the compounds which are specific for the desired target. This step therefore allows to retain leads which exhibit both the expected potency for the target and a good selectivity profile. Step (b) usually comprises determining the capacity of the selected hits to interact with, preferably to bind to various targets.

In a particular embodiment of this invention, step (b) comprises assaying the potent hits on more than 25 targets. In a more particular embodiment, said profiling step (b) comprises assaying the potent hits on more than 50 targets. In a most preferred embodiment, the profiling step (b) comprises assaying the potent hits on more than 60 targets. Illustrative examples of targets than can be assayed in step (b) are listed in Table I below. In a preferred embodiment of the invention, the profiling step (b) comprises screening the selected hits on the following targets, at least:
- non-peptidic G-coupled receptors, comprising an adenosine receptor, an adrenergic receptor, a dopamine receptor, a histamine receptor, a melatonin receptor, a muscarinic receptor, and a sigma receptor;
- peptidic G-coupled receptors, comprising an angiotensin receptor, a bombesin receptor, a bradykinin receptor, a cholecystokinin receptor, a chemokine receptor, an endothelin receptor, a galanin receptor, a neurokinin receptor, a neuropeptide Y receptor and a vasopressin receptor;
- an ion channel-coupled receptor, comprising a serotonin receptor ; and
- ion-channels, comprising a calcium-channel and a potassium channel.

Even more preferably, the assayed targets further comprise:
- enzymes, including a phosphodiesterase and a tyrosine kinase, and, optionally, a proteine kinase and a protease.

In a further preferred embodiment, the profiling step further comprises one or several cell-based assays, selected from :
- a cytokine secretion assay, such as a TNF-α secretion assay on human monocytes;
- a free radical assay, such as an 02 secretion assay on human granulocytes (e.g., HL60);
- an adhesion assay, in particular between human monocytes (e.g., U937 cells) and human endothelial cells;
- a cytotoxicity assay, on human monocytes for instance.

In a typical illustrative specific embodiment of the invention, the profiling step (b) comprises the screening of the compounds on each and every targets listed in Table III.

In another typical illustrative specific embodiment of the invention, the profiling step (b) comprises the screening of the compounds on each and every targets listed in Table I.

The capacity of the compounds to interact with or bind to these targets can be determined following the same methodology as described in step (a), in vitro or in cell-based systems. One important aspect of the invention is the ability to profile large numbers of compounds (potent hits) in order to produce improved leads over prior art methods. In this respect, to facilitate this profiling step (b), The applicant has developed a concept of High Throughpout Profiling ("HTP") which allows to quickly test in vitro numerous compounds (for instance in the range of one to two hundreds) in a large number of targets as disclosed above. More specifically, this HTP involves a robotic system which is able to run up to 20 protocols (one protocol per target) per day. This method is disclosed more in details in the examples. Basically, in a particular embodiment of the invention, the HTP is performed using a device comprising the following elements:
- working stations, including:
   . several incubators, more preferably 2 or 3
   . one or more filtration stations
   . reagent dispensers
- containers, preferably plates, and
- means to move the containers to the various working stations.

More specifically, the reagent dispensers allow the dispension of 10-20 targets and above 100 test compounds. The activity of the reagent dispensers can be manual or automated.

As discussed above, the plates used in the HTP step can be 96-, 384-, 864- or 1536-wells plates. Preferably, they contain 96, 384 or 864 wells.

The compounds retained after this step (b) are those which interact with the desired target and which do not interact with any other target or interact only with a few of them or poorly with other targets. The selectivity requirements can vary from one target to another and can be adapted by the skilled artisan. In particular, depending on the anticipated use of the compounds, interaction with several related targets (for instance, several serotoninergic receptors) can be tolerated, as long as the compound exhibit no interaction with other unrelated targets (such as histamin receptor, for instance). Step (b) of the present method is very important in that it determines the level of selectivity which is sought is the drug discovery process.

In a most preferred embodiment, the method according to this invention further comprises a high throughput lead development step, directly before or after step (b). In this regard, in a preferred embodiment of the present invention, the term profiling comprises the assessment of specificity as well as the assessment of pharmacological properties (i.e., the lead development step). This high throughput lead development ("HTLD") step preferentially comprises the screening of the hits for their pharmacological properties. More particularly, HTLD comprises screening the hits for the following properties:
- toxicity
- metabolism, and/or
- pharmacokinetic.

Toxicity is preferably determined by contacting the compounds with reporter cells, such as human monocytes for instance, and by measuring the release of a labelled reporter compound (i.e., Cr) according to known methods.

The metabolism is important in terms of predicting how the compound would behave in vivo. Determination of the metabolism of compounds can be performed in vitro or in cell-based assays, by contacting the compound with cytochrome(s) P450 and determining the properties of the resulting metabolites.

Pharmacokinetic is preferably assayed in a cellular model of the intestinal lumen, in particular on Caco-2 or MDCK cells.

In a particular variant of the method of the invention, step (i) therefore comprises:
(a) screening a library of compounds for activity against a desired target, to obtain potent hits ; and
(b) profiling the potent hits of step (a) for selectivity vis-a-vis the desired target and pharmacological properties, to obtain potent and selective leads.

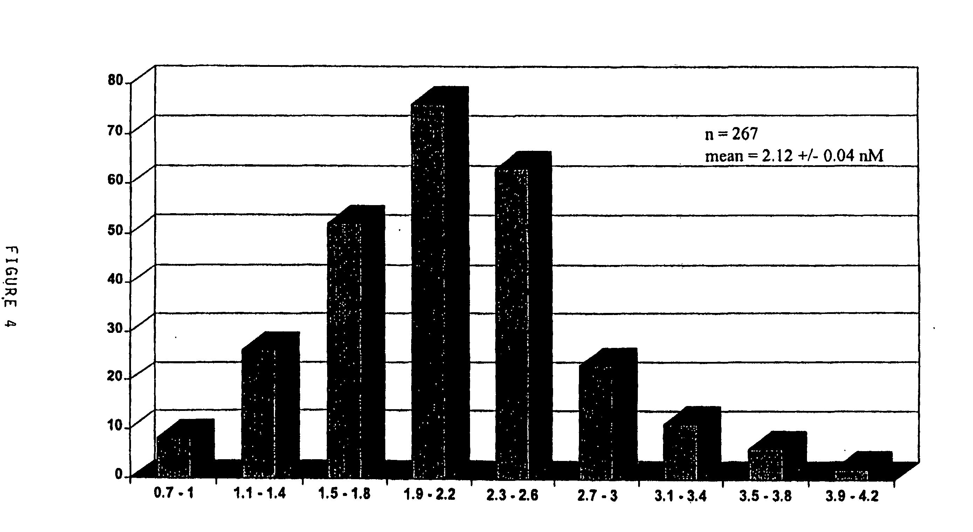

More preferably, step (b) comprises profiling the potent hits of step (a) for selectivity vis-a-vis the desired target and lack of toxicity on human cells in vitro. Even more preferably, step (b) comprises profiling the potent hits of step (a) for selectivity vis-a-vis the desired target, lack of toxicity on human cells in vitro and ability to cross a model of intestinal lumen. In a particularly advantageous embodiment, the compounds are tested also for their metabolism in the presence of cytochrome(s) P450. A schematic representation of such a method is given on Figure 3.

The leads thereby obtained can be used either directly in a drug candidate selection programm or in step (c) of the method, as templates to design and synthesize focused libraries of compounds structurally related thereto. These focused libraries can be produced according to the techniques disclosed below and known in the art, and may contain several thousands of compounds. The term "structurally related" indicates t hat these libraries are designed so that their compounds contain structures or motifs which are also present in the leads. For instance, these libraries may contain specific monomers or blocks identified in the leads, as disclosed in the examples. In particular, the construction of focused libraries generally involves, in a first step, the molecular characterization of the leads, in order to determine their constitutive blocks. Optionally, this initial step can also involves the clustering of the leads having common structural characteristics in order to synthezise a limited number of focused libraries. From this first analysis, the most common blocks and their analogs are used in a molecular modeling step, in order to create the focused library. The choice of the analogs can be made according to various, known analoging strategies, including analoging of monomers or analoging of the entire structure. This analoging can be performed, for instance, with appropriate softwares, by virtual screening of libraries. The libraries are then produced according to known combinatorial chemistry methods as disclosed below.

From these focused libraries, steps (a) to (c) can be repeated to obtain further focused libraries (step (ii)). Depending on the selectivity or potency which are sought, this cycle may be repeated from 0 to 10 times, preferably from 1 to 5 times.

These libraries are finally screened for the given target (step (iii)), to obtain active compounds. The screening of step (iii) can be carried out according to methods known in the art. Preferably, the screening of step (iii) is performed in a similar way as the screening and profiling of steps (a) and (b) above. This screening may also contain additional steps such as a genotoxicity assay or a functional assay as described in the examples.

The methods disclosed above can be applied to various libraries of compounds, including libraries of compounds of natural origin or obtained through well-known combinatorial chemistry methods. These combinatorial chemistry methods involve the synthesis of random or focused series of compounds starting from monomers or blocks, using for instance solid support- or liquid synthesis, parallel synthesis, microbeads, divide couple recombine or any other method known in the art (N.K. Terret, M. Gardner, D.W. Gordon, R.J. Kobylecki, J. Steele. "Combinatorial synthesis. The design of compound libraries and their application to drug discovery". Tetrahedron, Vol. 51, N° 30, (1995) pp 8135-8173). The compounds can therefore be nucleic acids, peptides, other chemicals or a combination thereof. These libraries may for instance comprise between 5 000 and 500 000 compounds, for instance between 10 000 and 20 000 compounds.

The methods of the invention can be used efficiently to identify various types of active compounds such as drug candidates, receptor ligands. The advantages of these methods are the possibility to produce leads which are both potent and selective for a target, thereby avoiding the follow-up of poor leads.

In this respect, the invention also relates to a method of High Throughput Profiling comprising the screening of a large number of compounds for their capacity to interact with one given target amongst a large number of targets. More preferably, the number of compounds tested is comprised between 5 and 500, more specifically between 5 and 100, such as for instance between 5 and 50. The number of targets assayed in preferably above 25, more prefarably above 50. It is particularly preferred to use a number of target above 60, such as for instance 62 or 73. An illustrative list of such targets is provided in Table I.
In a particular embodiment, the HTP method of this invention therefore comprises the screening of at least 50 compounds for their capacity to interact with one given target amongst at least 50 different targets.
In another preferred embodiment, the HTP method of this invention comprises the screening of at least 50 compounds for their capacity to interact with one given target amongst at least 50 different targets and for their pharmacological properties.
The screening of the compounds is usually performed by incubating each compound with each target, followed by the determination of an interaction (e.g. a binding). Such screening may be carried out for instance in microtitration plates, so as to perform as many screen as possible in a limited period of time. The invention disclosed in this respect a robotic system allowing this screening to be performed rapidly, such robotic system providing for an automated distribution of the compounds in the plates followed by a determination of the capacity of the compound to inhibit binding of ligand to the target. To carry out this method, the plates usually comprise the target coated to their surface, as well as a labelled ligand. As shown in the Examples, this method allows the determination, in a relatively short period of time, of various compounds on numerous targets (73).

The present invention also provides improved libraries of compounds. In particular, the invention provides focused libraries comprising a plurality of compounds, wherein said compounds have structural diversity and derive from compounds having the capacity to bind selectively to a common desired target. While prior art focused libraries comprise essentially compounds deriving from hits having the capacity to bind to a target, the invention now provides more defined libraries, which comprise compounds deriving from hits which have not only the capacity to bind to a target, but which are also specific for said target. These libraries are thus more focused than the previous ones and can be used as a source of compounds in a drug discovery program. The term "deriving" in this context means obtained by combinatorial chemistry methods based on the structure of such hits, according to methods known in the art.

The instant invention will be disclosed in more details in the following examples, which are only illustrative and in no way limit the scope of the instant invention.

### Legends to the Figures

Figure 1 : Classical drug discovery process
Figure 2 : Drug discovery process of the invention
Figure 3 : Drug discovery process of the invention
Figure 4 : Determination of the IC50 of selected hits for the rat mu receptor. Refence compound: DAGO.
Figure 5 : Structure of 12 leads.
Figure 6 : Determination of the affinity of the selected hits for the human mu receptor.
Figure 7 : HTP data for selected hits. The compounds selected from the affinity study have been tested on multiple targets. The identity of the targets is as described in Table I. The effect is indicated by the intensity of the grey in each case, white being inactive and black being the maximal effect. At the bottom of the figure, data from Table IV are reported, namely affinity values of the compounds for the opioid receptors.
Figure 8 : Functional assay: Determination of the inhibition of guinea-pig ileum contraction by the selected leads.

### Examples

### Example 1 : hit selection for the opioid mu receptor

A library of 10,000 compounds has been designed, synthesized and screened in a model of opioid mu receptors from rat origin using a classical binding assay system. More specifically, compounds were tested in solution, at a 10⁻⁵M concentration, in the presence of [³H] DAMGO as radioligand, on rat cortex homogenate. Specific binding was determined under competition with naloxone by liquid scintillation.

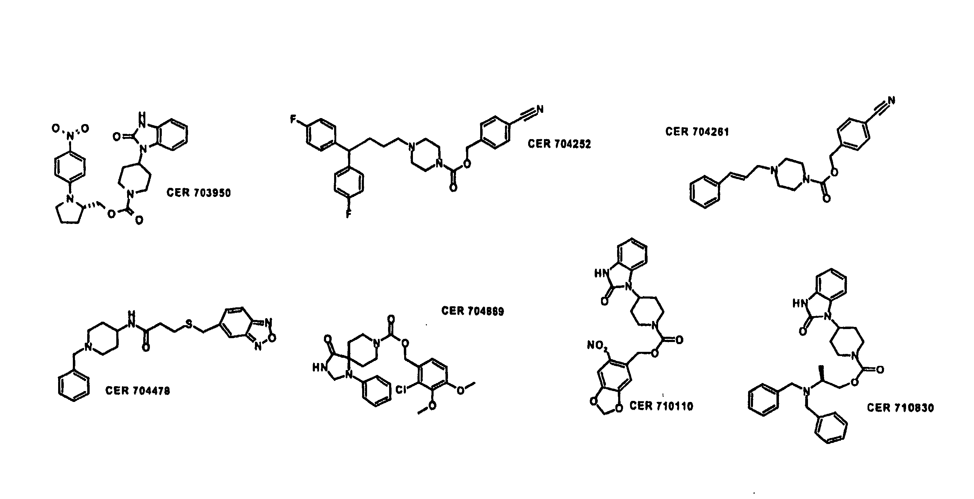

This primary screening resulted in numerous hits (Figure 4) : 180 compounds inhibited more than 50% the binding of the radioligand specific to the mu receptor. 111 inhibited more than 60% of said binding and 42 more than 70%. In order to obtain active compounds with a high potency, these latter 42 compounds have been used for the further steps.

The chemical structure of these 42 compounds have been analysed.

Among these 42 compounds, 24 were discarded because of their chemical structure (i.e. incompatibility with industrial applications) and 18 were retained for further analysis.

One of these compounds (CER680827) was not confirmed to be active in the rat mu assay. The 17 remaining compounds were re-synthesized according to methods known in the art, purified and their affinity was measured in human mu, delta and kappa receptor binding assays.

As shown in Table II, five compounds proved to be inactive (CER680435, CER728422, CER728428, CER829741, CER838941), either because the effect observed in the primary screening was due to a side product (primary screening is performed with unpurified compounds) or because of a species selectivity of these compounds (primary screening was performed using the rat receptor and affinity was determined using the human receptor).

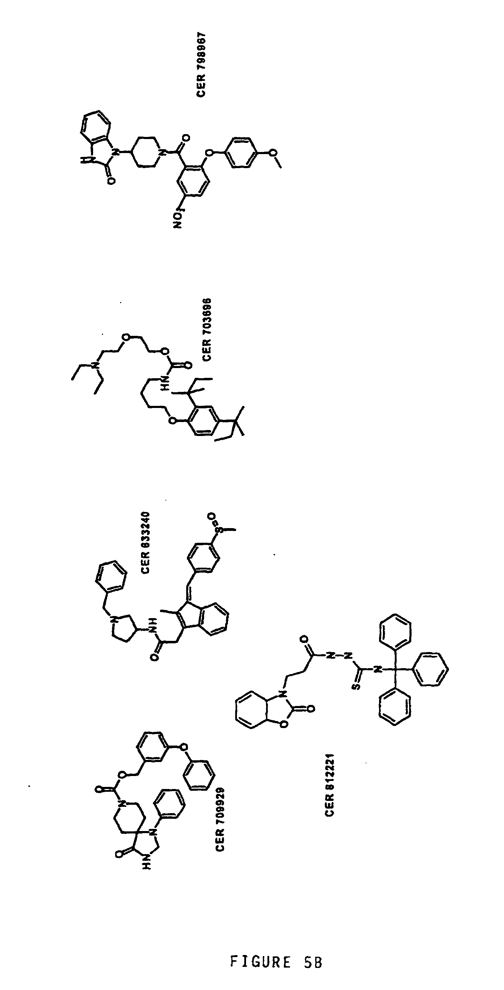

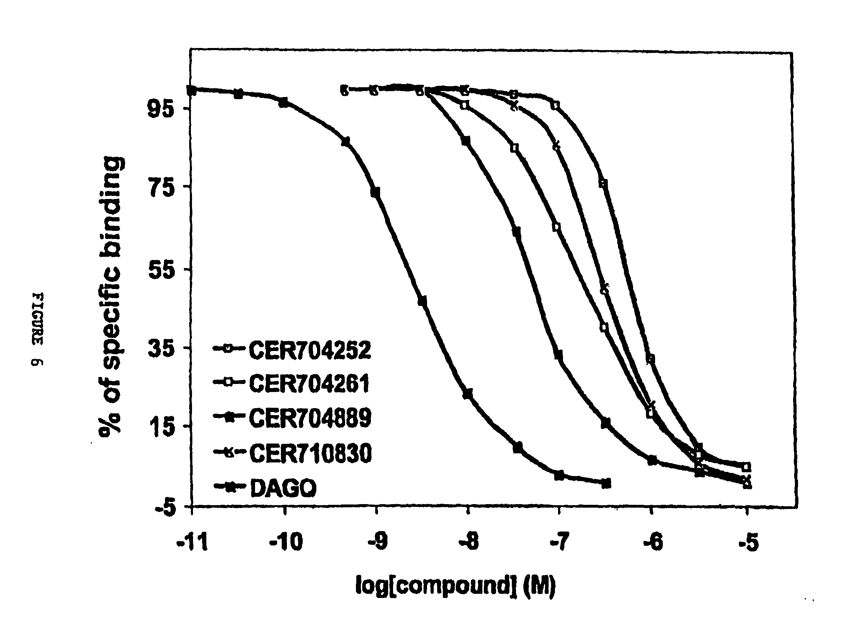

However, 12 hits exhibited the expected affinity for the mu receptor and only one of them (CER798967) was found to be more active on the delta receptor than on the mu receptor. The structure of these 12 compounds is represented on Figure 5. Data are summarized in Figure 6 and Table IV.

These 12 compounds have then been tested in the HTP system, i.e. their selectivity profile has been determined using 62 receptors as described in Table I. For that purpose, 10 µM of each compound is incubated in microtitration plates previously coated with the targets shown in Table I, in the presence of a reference labelled ligand specific for each of said target. Each compound is tested in duplicate.

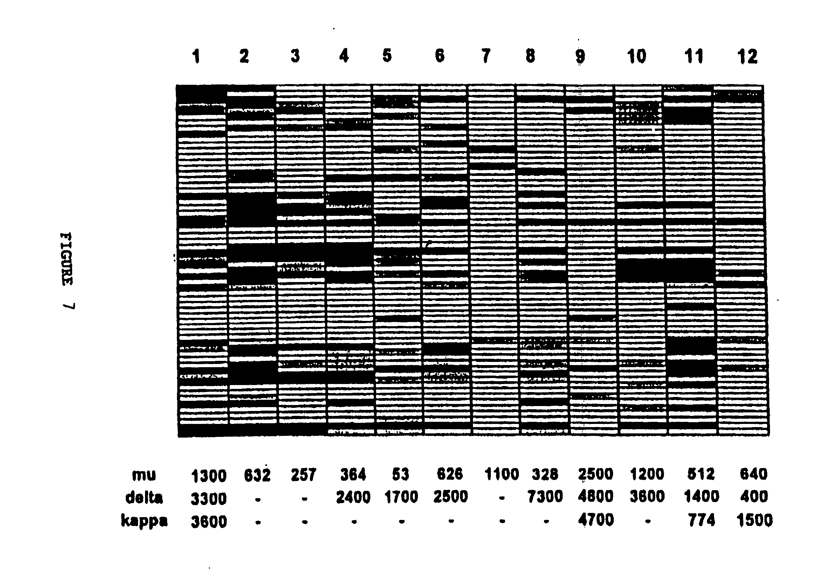

The results obtained are summarized in Figure 7. The HTP gave precious information since it allowed to show that some of the compounds which could have been selected on the basis of their relative affinity for opioid receptors proved to be active on many unrelated targets. These compounds have thus be discarded because of their unspecificity.

Combining the criteria of potency (affinity of the hits to the mu and other opioid receptors) and selectivity allowed to select 4 compounds (leads) as candidates for the follow-up of the lead optimization process. These compounds are CER704252, CER704261, CER704889 and CER710830.

In addition, in order to confirm the functionality of the lead compounds obtained according to the instant method, each of these compounds was tested for its capacity to inhibit guinea-pig ileum contraction as follows:

### Experimental procedures:

Ileum segments are obtained from male Dunkin Hartley guinea-pigs weighing 300-350 g. The tissues are suspended between two stainless-steel hooks in organ baths containing an oxygenated and pre-warmed physiological salt solution where they are connected to force-displacement transducers for isometric tension recording. Twitch contractions are elicited by field electrical stimulation (single pulses, 1 ms duration, maximal intensity, 0.1 Hz) delivered through two platinum electrodes by a multichannel constant current stimulator. The tissues are stretched to an optimal resting tension and then allowed to equilibrate for at least 30 min during which time they are washed repeatedly. Drugs are added after the twitch contraction amplitude is reproducible.

### Test for agonist activity:

The tissues are initially exposed to an effective concentration of the reference agonist DAMGO to verify tissue responsiveness. Following washout and another equilibration period, the tissues are exposed to several increasing concentrations of the test compounds or the same agonist. The different concentrations are added cumulatively and cach remains in contact with the tissues until a stable response is obtained. When an agonist like responses (inhibition of twitch contractions) is obtained, the reference antagonist naloxone is added after the action of the highest concentration of the test compounds to check the involvement of µ receptors in this response.

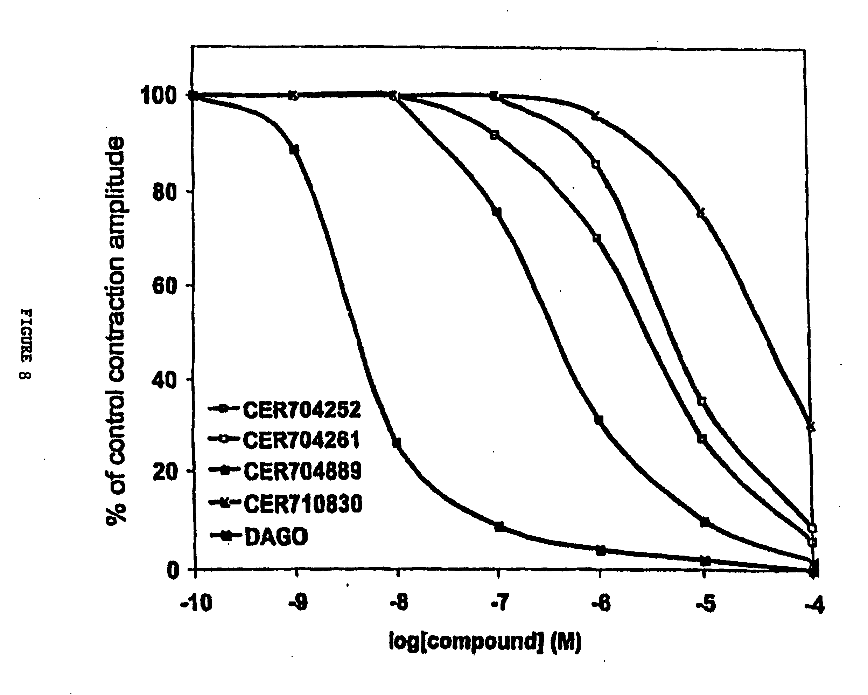

The results presented on Figure 8 confirm that the selected leads have all the capacity to inhibit guinea-pig ileum contraction which further demonstrates the reliability of the method of the invention.

All together, this first cycle of lead optimization has taken 6 weeks (synthesis, primary screening, confirmation, affinity to opioid receptors and HTP) and could be reduced to 3 weeks, mainly by decreasing the turnaround time of HTP. This will make the lead discovery both rapid and secure, in the sense that it will avoid the follow-up of poor lead candidates.

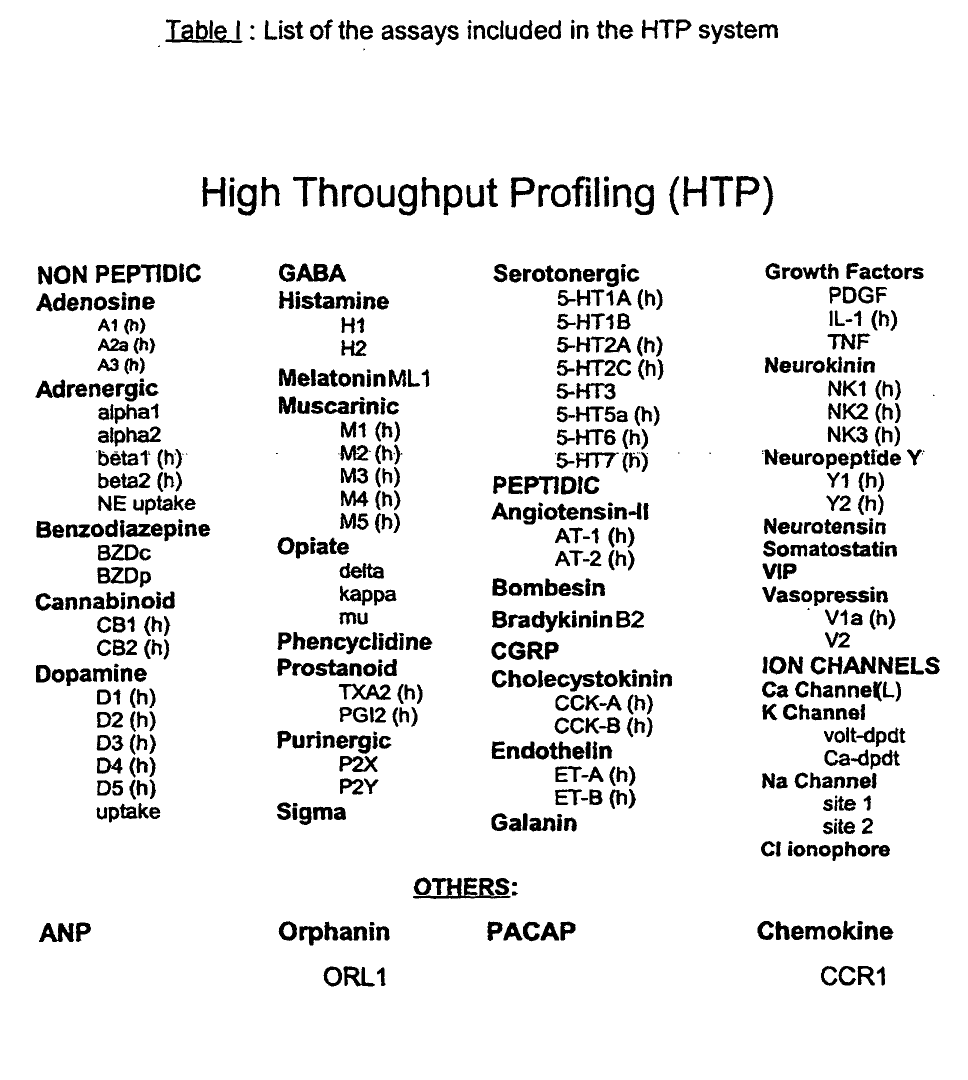

**Table II :**

| List of enzymes included in the HTP system | |
|---|---|
| Family | Name |
| Phosphodiesterases | PDE II (h) PDE IV(h) |
| | |
| Protein kinases | MAP kinase (h) PKc |
| | |
| Tyrosine kinases | EGF-tyr kinase (h) p56-tyr kinase |
| | |
| Proteases | Angiotensin-Convertin Enzyme (ACE) (h) Cathepsin B (h) Elastase (h) |

| | |
|---|---|
| (h) indicates that the assay is performed with an enzyme of human origin | |

**Table III :**

| Typical list of assays for HTP system **G PROTEINS COUPLED RECEPTORS** | |
|---|---|
| **Non-peptidic Receptors** | |
| Adenosine | A₁ (h) |
| Adrenergics | α₁ |
| | α2 |
| | β1 |
| | β2 |
| Dopamine | D₁ (h) |
| | D₂ (h) |
| Histamine | H₁ |
| melatonin | ML1 |
| Muscarinics | M₁ (h) |
| | M₃ (h) |
| Opiates | mu (h) |
| Serotonine | 5-HT_{1A} (h) |
| | 5-HT_{1D} |
| | 5-HT_{2C} (h) |
| | 5-HT₆ (h) |
| Sigma | σ1 |

| **Peptidic Receptors** | |
|---|---|
| Angiotensin | AT1 (h) |
| Bombesin | BB |
| Bradykinin | B₂ |
| CGRP | CGRP (h) |
| Cholecystokinin | CCK_{A} (h) |
| Chemokine and Cytokine | IL-1β |
| | CCR₂ (h) |
| Endothelin | ETA (h) |
| Galanin | Galanine (h) |
| Glucagon-like peptide | GLP-1 (h) |
| Neurokinin | NK₁ (h) |
| Neuropeptide Y | Y₁ (h) |
| Vasopressine | V_{1A} (h) |
| | |

| **ION CHANNEL-COUPLED RECEPTORS** | |
|---|---|
| Benzodiazepin central | BZDc |
| Serotonine | 5-HT3 |
| | |

| **CARRIERS** | |
|---|---|
| Norepinephrine | NE uptake (h) |
| Dopamine | DA uptake (h) |
| Serotonine | 5-HT uptake |

| **ION CHANNELS** | |
|---|---|
| Calcium Channel | Ca (verapamil) |
| Potassium Channel | K (ATP dependant) |
| Sodium Channel | Na (site 2) |
| Chloride Channel | Cl ionophore |

| **ENZYMES** | |
|---|---|
| Phosphodiesterases | PDE II (h) |
| | PDE IV (h) |
| Proteine kinases | MAP kinase (h) |
| | Pk_{c} |
| Tyrosine kinases | EGF-tyr kinase (h) |
| | p56-tyr kinase |
| Proteases | Angiotensin-Converting Enzyme (ACE) (h) |
| | Cathepsine B (h) |
| | Elastase (h) |

| CELL-BASED ASSAYS | |
|---|---|
| Cytokine | TNF-α secretion by human monocytes |
| Free radical | O₂ secretion by human granulocytes (HL60) |
| Adhesion | of human monocytes (U937) on human endothelial cells |
| Cytotoxicity | of human monocytes |
| (h : test performed on human proteins) | |

**Table IV :**

| Affinity of hits on the opioid receptors | | | | | | | |
|---|---|---|---|---|---|---|---|
| **Compound** | **mu** | **delta** | **kappa** | **Compound** | **mu** | **delta** | **kappa** |
| **CER680435** | **-** | **-** | **-** | **CER710830** | **328** | **7300** | **-** |
| **CER680827** | **-** | **-** | **-** | **CER728422** | **-** | **-** | **-** |
| **CER703950** | **1300** | **3300** | **3600** | **CER728428** | **-** | **-** | **-** |
| **CER704252** | **632** | **-** | **-** | **CER812221** | **2500** | **4800** | **4700** |
| **CER704261** | **257** | **-** | **-** | **CER829741** | **-** | **8400** | **-** |
| **CER704478** | **364** | **2400** | **-** | **CER838941** | **-** | **-** | **-** |
| **CER704889** | **53** | **1700** | **-** | **CER833240** | **1200** | **3600** | **-** |
| **CER709929** | **626** | **2500** | **10000** | **CER703696** | **512** | **1400** | **774** |
| **CER710110** | **1100** | **-** | **-** | **CER798967** | **640** | **400** | **1500** |
| *Affinities of compounds are given in nM. The sign - indicates the absence of effects in the concentration range tested (0.1* to *10,000 nM)* | | | | | | | |

## Claims

1. A high throughput screening method for the identification of leads from a library of compounds, said method comprising the steps of :
(a) screening a library of compounds for activity against a desired target, to obtain potent hits;
(b) profiling the potent hits of step (a) on more than 25 targets comprising at least:
- non-peptidic G-coupled receptors,
- peptidic G-coupted receptors,
- an ion channel-coupled receptor, and
- ion-channels,
to obtain potent hits with a selected profile.

2. A method of identification of active compounds from a library of compounds, said method comprising:
(i) a high throughput screening comprising the steps of :
(a) screening a library of compounds for activity against a desired target, to obtain potent hits;
(b) profiting the potent hits of step (a) to obtain potent and selective leads;
(c) preparing focused library(ies) of compounds structurally related to the leads of step (b);
(ii) repeating, one or several times, step (i) using the focused library(ies) of step (c) to generate further focused libraries, and,
(iii) screening and profiling the focused libraries of step (i) or (ii) for activity against said target, to obtain active and specific compounds.

3. Method of claim 1 or 2 wherein said screening of step (a) comprises the selection of compounds capable of interacting to a desired target.

4. Method of claim 2 wherein said screening of step (a) further comprises measuring the affinity of the compounds capable of binding to said desired target, in order to select, as potent hits, the compounds having the best affinity.

5. Method of claim 1 or 2 wherein said profiling step (b) comprises assaying the potent hits on said targets and selecting the compounds which are specific for the desired target.

6. Method of claim 5 wherein said profiling step (b) comprises assaying the potent hits on more than 50 targets.

7. Method of claim 6 wherein said profiting step (b) comprises assaying the potent hits on more than 70 targets.

8. Method according to any of the preceding claims, wherein step (i) comprises:
(a) screening a library of compounds for activity against a desired target, to obtain potent hits ; and
(b) profiling the potent hits of step (a) for selectivity vis-a-vis the desired target and pharmacological properties, to obtain potent and selective leads.

9. Method of claim 1, wherein, in step (b), between 5 to 500 potent hits are profiled.

10. Method of claim 1 or 2 wherein said library of compounds comprises between 5 000 and 500 000 compounds.

11. Method of claim 10 wherein said library of compounds comprises between 10 000 and 20 000 compounds.

12. Method of claim 2 wherein said active compounds are drug candidates.

13. Method of claim 2 wherein said active compounds are receptor ligands.

## Patentansprüche

1. Hochdurchsatz-Durchmusterungsverfahren zur Identifizierung von Führungssubstanzen aus einer Verbindungsbank, wobei das Verfahren die Schritte umfasst:
(a) Durchmusterung einer Verbindungsbank auf Aktivität gegen eine erwünschte Zielstruktur, um wirkungsvolle Treffer zu erlangen;
(b) Profilbestimmung der wirkungsvollen Treffer aus Schritt (a) an mehr als 25 Zielstrukturen, wenigstens umfassend:
- nicht-peptidische G-gekoppelte Rezeptoren,
- peptidische G-gekoppelte Rezeptoren,
- ein Ionenkanal-gekoppelter Rezeptor, und
- Ionenkanäle,
um so wirkungsvolle Treffer mit einem ausgewählten Profil zu erhalten.

2. Verfahren für die Identifizierung aktiver Verbindungen aus einer Verbindungsbank, wobei das Verfahren die Schritte umfasst:
(i) eine Hochdurchsatz-Durchmusterung, umfassend die Schritte:
(a) Durchmusterung einer Verbindungsbank auf Aktivität gegen eine erwünschte Zielstruktur, um so wirkungsvolle Treffer zu erhalten;
(b) Profilbestimmung der wirkungsvollen Treffer aus Schritt (a), um so wirkungsvolle und selektive Führungssubstanzen zu erhalten;
(c) Herstellung (einer) fokussierter(n) Bank(en) von Verbindungen, die strukturell mit den Führungssubstanzen aus Schritt (b) verwandt sind;
(ii) ein- oder mehrmalige Wiederholung von Schritt (i) unter Verwendung der fokussierten Bank(en) aus Schritt (c), um so weiter fokussierte Banken herzustellen, und
(iii) Durchmusterung und Profilbestimmung der fokussierten Banken aus Schritt (i) oder (ii) auf Aktivität gegen die Zielstruktur, um so aktive und spezifische Verbindungen zu erhalten.

3. Verfahren gemäß Anspruch 1 oder 2, wobei die Durchmusterung aus Schritt (a) die Auswahl von Verbindungen umfasst, die in der Lage sind, mit einer erwünschten Zielstruktur zu interagieren.

4. Verfahren gemäß Anspruch 2, wobei die Durchmusterung aus Schritt (a) weiterhin die Affinitätsmessung der Verbindungen umfasst, die in der Lage sind, an die erwünschte Zielstruktur zu binden, um so die Verbindungen mit der besten Affinität als wirkungsvolle Treffer auszuwählen.

5. Verfahren gemäß Anspruch 1 oder 2, wobei die Profilbestimmung aus Schritt (b) die Untersuchung der wirkungsvollen Treffer an den Zielstrukturen und die Auswahl der Verbindungen umfasst, die spezifisch für die erwünschte Zielstruktur sind.

6. Verfahren gemäß Anspruch 5, wobei die Profilbestimmung aus Schritt (b) die Untersuchung der wirkungsvollen Treffer an mehr als 50 Zielstrukturen umfasst.

7. Verfahren gemäß Anspruch 6, wobei die Profilbestimmung aus Schritt (b) die Untersuchung der wirkungsvollen Treffer an mehr als 70 Zielstrukturen umfasst.

8. Verfahren gemäß einem der vorhergehenden Ansprüche, wobei der Schritt (i) umfasst:
(a) Durchmusterung einer Verbindungsbank auf Aktivität gegen eine erwünschte Zielstruktur, um wirkungsvolle Treffer zu erlangen; und
(b) Profilbestimmung der wirkungsvollen Treffer aus Schritt (a) auf Selektivität für die erwünschte Zielstruktur und ihrer pharmakologischen Eigenschaften, um so wirkungsvolle und selektive Führungssubstanzen zu erhalten.

9. Verfahren gemäß Anspruch 1, wobei in Schritt (b) für zwischen 5 bis 500 wirkungsvolle Treffer das Profil bestimmt wird.

10. Verfahren gemäß Anspruch 1 oder 2, wobei die Verbindungsbank zwischen 5.000 und 500.000 Verbindungen umfasst.

11. Verfahren gemäß Anspruch 10, wobei die Verbindungsbank zwischen 10.000 und 20.000 Verbindungen umfasst.

12. Verfahren gemäß Anspruch 2, wobei die aktiven Verbindungen Kandidaten für Arzneimittel sind.

13. Verfahren gemäß Anspruch 2, wobei die aktiven Verbindungen Rezeptorliganden sind.

## Revendications

1. Procédé de criblage à haut débit pour identifier des composés têtes de série à partir d'une bibliothèque de composés, ledit procédé comprenant les étapes de :
(a) criblage d'une bibliothèque de composés pour une activité contre une cible désirée afin d'obtenir des candidats puissants ;
(b) obtention d'un profil des candidats puissants de l'étape (a) sur plus de 25 cibles comprenant au moins :
- des récepteurs couplés à G non peptidiques,
- des récepteurs couplés à G peptidiques,
- un récepteur couplé à un canal ionique, et
- des canaux ioniques,
pour obtenir des candidats puissants avec un profil sélectionné.

2. Procédé d'identification de composés actifs à partir d'une bibliothèque de composés, ledit procédé comprenant :
(i) un criblage à haut débit comprenant les étapes de :
a. criblage d'une bibliothèque de composés pour une activité contre une cible désirée, afin d'obtenir des candidats puissants ;
b. obtention d'un profil des candidats puissants de l'étape (a) pour obtenir des composés têtes de série puissants et sélectifs ;
c. préparation de bibliothèque(s) focalisée(s) de composés structurellement liés aux composés têtes de série de l'étape (b) ;
(ii) répétition, une ou plusieurs fois, de l'étape (i) en utilisant la (ou les) bibliothèque(s) de l'étape (c) pour générer d'autres bibliothèques focalisées, et,
(iii)criblage et obtention de profils des bibliothèques focalisées de l'étape (i) ou (ii) pour l'activité contre ladite cible, afin d'obtenir des composés actifs et spécifiques.

3. Procédé selon la revendication 1 ou 2, dans lequel ledit criblage de l'étape (a) comprend la sélection de composés capables d'interagir avec une cible désirée.

4. Procédé selon la revendication 2, dans lequel ledit criblage de l'étape (a) comprend en outre une étape de mesure de l'affinité des composés capables de se lier à ladite cible désirée, afin de sélectionner, comme candidats puissants, les composés ayant la meilleure affinité.

5. Procédé selon la revendication 1 ou 2, dans lequel ladite étape (b) de profil comprend le dosage des candidats puissants sur lesdites cibles et la sélection des composés qui sont spécifiques pour la cible désirée.

6. Procédé selon la revendication 5, dans lequel ladite étape (b) de profil comprend le dosage des candidats puissants sur plus de 50 cibles.

7. Procédé selon la revendication 6, dans lequel ladite étape (b) de profil comprend le dosage des candidats puissants sur plus de 70 cibles.

8. Procédé selon l'une quelconque des revendications précédentes, dans lequel l'étape (i) comprend :
(a) le criblage d'une bibliothèque de composés pour une activité contre une cible désirée, pour obtenir des candidats puissants ; et
(b) l'obtention d'un profil des candidats puissants de l'étape (a) pour la sélectivité vis-à-vis de la cible désirée et des propriétés pharmacologiques, pour obtenir des composés têtes de série puissants et sélectifs.

9. Procédé selon la revendication 1, dans lequel, dans l'étape (b), on obtient le profil de 5 à 500 candidats puissants.

10. Procédé selon la revendication 1 ou 2, dans lequel ladite bibliothèque de composés comprend entre 5000 et 500000 composés.

11. Procédé selon la revendication 10, dans lequel ladite bibliothèque de composés comprend entre 10000 et 20000 composés.

12. Procédé selon la revendication 2, dans lequel lesdits composés actifs sont des candidats médicaments.

13. Procédé selon la revendication 2, dans lequel lesdits composés actifs sont des ligands de récepteurs.
